# EUROPEAN PATENT SPECIFICATION

(11) **EP 4 642 409 B1**
(45) Date of publication and mention of the grant of the patent: **25.11.2026**
(21) Application number: 22846910.2
(22) Date of filing: 27.12.2022
(51) Int. Cl.: A61F 13/02

(54) **METHOD FOR MANUFACTURING MEDICAL DRESSINGS**
VERFAHREN ZUR HERSTELLUNG VON MEDIZINISCHEN VERBÄNDEN
PROCÉDÉ DE FABRICATION DE PANSEMENTS MÉDICAUX

(43) Date of publication of application: 05.11.2025
(73) Proprietor: Mölnlycke Health Care AB, 431 21 Mölndal (SE)
(72) Inventor: WATHNE, Christian, 435 30 MÖLNLYCKE (SE); MELIN, Daniel, 435 30 MÖLNLYCKE (SE); JAKOBSSON, Conny, 443 38 LERUM (SE); JOUSINEN, Erkki, 50170 MIKKELI (FI)
(74) Representative: Kransell & Wennborg KB
(86) International application number: PCT/EP2022/087872
(87) International publication number: WO 2024/141151

(56) References cited:
- EP-A1- 3 932 374
- US-A1- 2021 378 876

## Description

### TECHNICAL FIELD

The present disclosure generally relates to a method for manufacturing medical dressings. More specifically, the present disclosure relates to a method for manufacturing medical dressing with medical dressing specific markings.

### BACKGROUND

Generally, manufacturing of medical dressing comprises processing of various continuous material webs, which are fed out from rolls or the like, combined with other continuous material webs, thus forming a web assembly, and passed through different workstations for carrying out different process steps. Such manufacturing of medical dressings may include providing a first material web intended to form a backing layer of the medical dressing consisting of a plastic film, a second absorbent layer web, and an adhesive skin contact layer web intended to form a wound facing side of the medical dressing. The manufacturing may also include application of one or more webs of release liner sheet(s) to protect the adhesive skin contact layer from contamination prior to use. Conventionally, the release liner is applied as two or more parallel release liner sheets which corresponds in width to the final medical dressing, i.e. are thus not coextensive with the medical dressing web to which they are applied. In a final step, the medical dressings are cut out from the web assembly, in certain cases this may include contour cutting the medical dressings out from the web assembly. Relevant prior art is represented by EP3932374 and by US2021378876.

Adhesive medical dressings are frequently used in wound care, both for the purpose of treating wounds and scars and for the purpose of preventing these from occurring in the first place. Certain types of medical dressings may require instructions, such as cutting of the medical dressing or how to properly apply the medical dressings to the patient. In view of this, the medical dressings, and more specifically the release liner, may be provided with medical dressing specific markings, communicating instructions to caregivers or medical staff relating to a correct use and application of the dressings.

Manufacturing of medical dressings is carried out at high speed and often with a plurality of medical dressings arranged in parallel, as seen in a Machine direction (MD), being cut out simultaneously. When providing parallel release liner sheets with medical dressing specific markings the release liner sheets need to be correctly positioned onto the underlying medical dressing web to ensure alignment of the medical dressing specific markings prior to cutting. Otherwise, there may be a risk that the medical dressing specific markings are arranged slightly off set, as seen in the CD direction. Furthermore, tensions in the assembly web leading to varying and unpredictable distances between the medical dressing specific markings, as seen in a Machine Direction (MD), may also lead to difficulties when cutting out the medical dressings and a risk for an offset position of the markings as seen in the MD direction on the cut out medical dressing.

In view of the above, there is a need to provide an improved manufacturing method for medical dressings comprising medical dressing specific markings.

### SUMMARY

It is an object of the present disclosure to make available an improved and efficient method for manufacturing medical dressings comprising medical dressing specific markings that at least partially overcome the above-described deficiencies. This is achieved by a method for manufacturing medical dressings comprising medical dressing specific markings according to claim 1.

Hence, according to a first aspect, there is provided a method for manufacturing medical dressings comprising medical dressing specific markings comprising the steps of;
- feeding a continuous medical dressing web in a Machine Direction (MD), the medical dressing web comprising a backing layer, an absorbent layer and an adhesive skin contact layer, the absorbent layer being arranged between the backing layer and the adhesive skin contact layer, the continuous medical dressing web having a first and a second longitudinal edge extending in the Machine Direction (MD) and a first and a second longitudinal edge region extending along the first and the second longitudinal edge of the continuous medical dressing web;
- providing a release liner web, the release liner web being provided with a plurality of medical dressing specific markings arranged in a first and second row extending in a Cross machine Direction (CD), thereby defining a first and a second row of medical dressing substrates comprising a respective medical dressing specific marking, each of the first and a second row of medical dressing substrates being arranged between the first and the second longitudinal edge region of the medical dressing web, the release liner web furthermore being provided with a continuous or discontinuous CD synchronization mark(s) extending along the first and/or second longitudinal edge of the continuous medical dressing web, the release liner web furthermore being provided with a respective MD synchronization mark for the first and the second row of medical dressing substrates;
- detecting the CD synchronization mark(s) on the release line web to align the release liner web and the medical dressing web, as seen in the CD direction;
- arranging the release liner web on the adhesive skin contact layer;
- detecting the respective MD synchronization mark and positioning of a contour cutting tool at a predetermined position with respect to the first and the second rows of medical dressing substrates, defined by the first and second row of medical dressing specific markings, based on the detected respective MD synchronization mark; and
- cutting out individual medical dressings from the continuous medical dressing web.

The medical dressing specific markings provided on the release liner web may guide the caregivers and medical staff to a correct use or application of the dressing.

Since the process is a high-speed process including several steps, such as applying a release liner web on a medical dressing web, including the backing layer, the absorbent layer and an adhesive skin contacting layer and furthermore contour cutting multiple of medical dressings from the web assembly, it is critical both that the release liner web is correctly positioned in relation to the medical dressing web and that the cutting tool is correctly positioned with respect to the plurality of medical dressing specific markings prior to cutting out of the individual medical dressings, one row at a time, such that the cut out medical dressing shape is aligned with a respective medical dressing specific marking.

The first and the second row of medical dressing specific markings extending in the CD direction, and the first and second row of medical dressing substrates defined by the first and the second row of medical dressing specific markings, are thus arranged in parallel, as seen in the MD direction. Each of the rows may comprise two, three, four, five or six medical dressing specific markings and thus two, three, four, five or six medical dressing substrates arranged in parallel, as seen in the MD direction.

The present method illustrates a continuous method, and the release liner web is a continuous release liner web and may be provided with a continuous number of rows, with medical dressing specific markings extending in the CD direction, thereby defining a continuous number of rows of medical dressing substrates being arranged between the first and the second longitudinal edge region of the continuous medical dressing web. The release liner web may thus furthermore be provided with a respective MD synchronization mark for each row of medical dressing substrates as disclosed herein for the first and the second row.

In the cutting step, the first row of medical dressing substrate is first cut out simultaneously, followed by the second row of medical dressing substrates being cut out simultaneously in a subsequent cutting step, i.e., one row at a time. In a continuous method, each row of medical dressing substrates, as seen in the CD direction, are thus cut out row by row, as seen in the CD direction.

The present disclosure is at least partially based on the realization that the provision of a release liner web provided with rows of medical dressing specific markings, as seen in the CD direction, allowing cutting a plurality of medical dressings provided with markings simultaneously, facilitates steering and positioning of the release liner web in relation to the medical dressing web and predetermining the position of the first and the second medical dressing specific markings, as seen in a CD direction. The fact that the release liner web is provided with rows of medical dressing specific markings extending in the CD direction and that the release liner web is provided with CD synchronization mark (s) enables reading of the position of the entire release liner web to correctly position the release liner web onto the adhesive skin contacting layer such that it is aligned with the medical dressing web, as seen in the CD direction. The position of the release liner web and/or the medical dressing web may subsequently be steered and adjusted, if needed, after reading of the CD synchronization mark (s) such that the release liner web is aligned with the medical dressing web.

The continuous or discontinuous CD synchronization mark may have a width, as seen in the CD direction of from 10 mm to 20 mm. The CD synchronization mark is preferably continuous along the first and/or second longitudinal edge of the continuous medical dressing web to enable continuous guiding of the release liner web to align the medical dressing specific markings provided on the release liner web in relation to a cutting tool.

The release liner web is furthermore provided with a respective MD synchronization mark for each row of medical dressing substrate, which is defined by a respective medical dressing specific marking provided on the release liner web, to provide indication of the position of the rows of the medical dressing substrates, as defined by the medical dressing specific markings, to correctly determine and set the position of the cutting tool.

The term "medical dressing substrate" is used to describe the portion of the medical dressing web which is intended to form the final medical dressing after being cut out at the contour cutting station. The position of the medical dressing substrate is determined by the position of the medical dressing specific markings.

The release liner web may be essentially co-extensive with the medical dressing web in the CD direction. This means that the webs have the same width or that the width of the release liner web does not differ from the width of the medical dressing web with more than 5%, such as not more than 3%, or not more than 1% compared in the CD direction. In general, the skin adhesive contacting layer has a somewhat narrower width than the release liner web to avoid problems in production caused by the stickiness of the skin adhesive contacting layer. Preferably, the skin adhesive contacting layer has a somewhat narrower width than the absorbent layer and the backing layer. Hence, the width of the release liner web may be from 5 mm to 40 mm wider than the skin adhesive contacting layer, such as from 5 mm to 20 mm wider than the skin adhesive contacting layer.

The medical dressing substrates of the first and the second row may each have a length, as measured in the MD direction and wherein the MD synchronization marks each has a frontmost edge, as seen in the MD direction, and wherein a distance between the frontmost edge of the MD synchronization mark of the first row and the frontmost edge of the MD synchronization mark of the second row, as measured in the MD direction, is the same as the length of the medical dressing substrates of the first and the second row. This may provide indication of the position of the rows of the medical dressing substrate to correctly determine and set the position of the cutting tool. The contour of the medical dressing substrate may be indicated by a contour marking provided on the release liner web or may be predetermined only in relation to the position of the medical dressing specific markings.

The respective MD synchronization marks may have a width, as seen in the CD direction, within the range of from 5 mm to 20 mm and a length, as seen in the MD direction, within the range of from 10 mm to 40 mm.

Each of the medical dressing substrates of the first and the second row may have the same length.

The method for manufacturing medical dressings according to the present disclosure may comprise a step of;
- arranging one or more release liner strips onto the adhesive skin contact layer; and in a subsequent step,
- arranging the release liner web on top the one or more release liner strips and on the adhesive skin contact layer.

The step of arranging one or more release liner strips may include arranging one or more continuous release liner strip webs onto the adhesive skin contact layer.

The one or more release liner strips may be folded release liner strip(s) comprising a respective folding line extending in the MD direction and overlapping with each of the medical dressing substrates in the respective first and second row.

Medical dressings may be provided with overlapping release liner strips to facilitate removal of the release liner from the medical dressing and also to facilitate positioning of the medical dressing by leaving a release liner strip on an edge portion of the medical dressing while positioning the medical dressing correctly over the skin or wound of the patient.

The release liner strip(s) may be applied in conjunction with the release liner web, for example, such that one or more release liner strips are applied in the same step to the medical dressing step. The release liner strip(s) are continuous strips of material having a narrower width compared to the release liner web.

The release liner web and the release liner strips may be formed from different materials.

A conventional release liner is typically formed from a first folded release liner portion and a second release liner portion overlapping the folded first release liner portion. The slight overlap between the folded release and the overlying release liner forms a "tab" that the wearer or caregiver can grasp to facilitate removal.

The release liner web of the present disclosure may be somewhat more difficult to grasp than a conventional release liner since it is co-extensive with the adhesive skin contact layer. The difficulties in grasping and removing the release liner web from the release liner strips are particularly enhanced if the release liner web and the release liner strips are formed from the same materials. During manufacturing of the dressing, and particularly during assembly of the dressing components, the release liner web and the release liner strips tend to adhere to each other, particularly at the interface between the release liner web and the release liner strips, which, in the present case, is relatively large. One reason for such undesired adherence may be that heat generated from friction during assembly of the dressing components can cause the release liners to become partially welded or fused together. Hence, different materials with different melting points are advantageously utilized for the release liner web and the release liner strips. This facilitates the removal of the release liner web from the underlying release liner strip (and from the dressing).

The release liner web may comprise a first polymeric film, and wherein the release liner strips comprise a second polymeric film, wherein the first polymeric film is different from said second polymeric film.

The first and/or said second polymeric film may be selected from a polyethylene film, a polypropylene film and a polyurethane film.

In the method, the first transverse edges of the medical dressing substrates may correspond to the frontmost edges of the medical dressing substrates, as seen in the MD direction, and wherein the MD synchronization marks may be aligned with first transverse edges of the medical dressing substrates in the respective first and second row.

The MD and CD synchronization marks may be arranged in the first and/or second longitudinal edge region of the continuous medical dressing web, preferably the MD and CD synchronization marks are arranged in the same longitudinal edge region.

The medical dressing specific markings may be individual instructions indicating how to cut, fold, use or apply said dressing.

The medical dressing specific markings may be formed by a print pattern, a weld pattern, a compressed pattern, an embossed pattern, a material deposition pattern, or combinations thereof.

Each of the medical dressing substrates may have a transverse extension and a longitudinal extension, wherein each of the medical dressing substrates may comprises a transverse center line dividing the each of the medical dressing substrates into a first portion and a second portion; the first and the second portion being symmetric about the a transverse center line, wherein the first portion comprises a first set of markings, and wherein the second portion comprises a second set of markings, and wherein the first set of markings is different from the second set of markings.

### BRIEF DESCRIPTION OF THE DRAWINGS

The various aspects of the present disclosure, including its particular features and advantages, will be readily understood from the following detailed description and the accompanying drawings, in which:
Fig. 1a illustrates a section of a continuous medical dressing web including a release liner web, according to the present disclosure.
Fig. 1b illustrates the section of the continuous medical dressing web illustrated in Fig. 1a with the release liner web being separated from the continuous medical dressing web.
Fig. 2A illustrates a first step in the method of manufacturing medical dressings of feeding a continuous medical dressing web in a Machine Direction (MD), the medical dressing web comprising a backing layer, an absorbent layer and an adhesive skin contact layer.
Fig. 2B illustrates a step in the method of manufacturing medical dressings according to the present disclosure of arranging release liner strips on top of the adhesive skin contact layer, according to an exemplary embodiment of the present disclosure.
Fig. 2C illustrates the steps in the method of manufacturing medical dressings according to the present disclosure of detecting a CD synchronization mark provided on the release liner web for correct positioning of the release liner web in relation to the medical dressing web and arranging the release liner web on top of the release liner strips and the adhesive skin contact layer of the continuous medical dressing web, according to an exemplary embodiment of the present disclosure.
Fig. 2D illustrates the steps in the method of manufacturing medical dressings according to the present disclosure of conveying the continuous medical dressing web towards a contour cutting station, detecting MD synchronization marks provided on the release liner web for positioning of a cutting tool in a predetermined position, and align and fit the cut-out medical dressings with the layout of the medical dressing specific markings.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

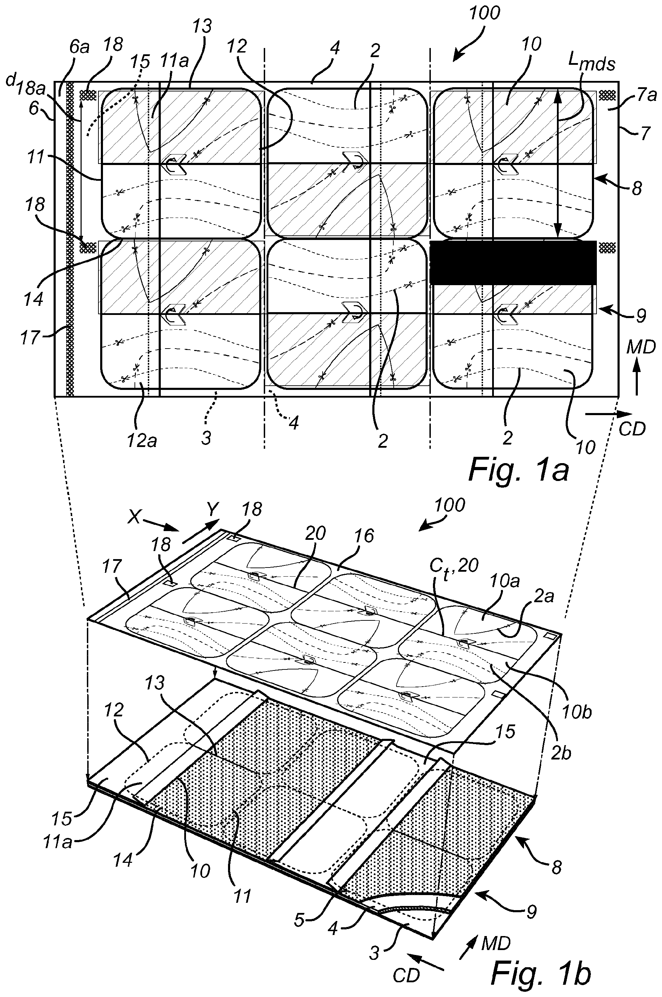

With reference to figures 1a and 1b, a section of a continuous medical dressing web 100 according to the present disclosure is conceptually illustrated. The medical dressing web 100 extends in a Machine Direction MD and a Cross Machine direction CD. The medical dressing web 100 comprises a backing layer 3, an absorbent layer 4 and an adhesive skin contact layer 5, the absorbent layer 4 being arranged between the backing layer 3 and the adhesive skin contact layer 5. The continuous medical dressing web 100 has a first and a second longitudinal edge 6,7 extending in the MD direction and a first and a second longitudinal edge region 6a,7a extending along the first and the second longitudinal edge 6,7 of the continuous medical dressing web 100.

Release liner strips 15 are arranged on top of the adhesive skin contact layer 5. The release liner strips 15 may be configured to cover from 20 to 50 %, e.g., from 30 to 40 % of the surface area of the adhesive skin contact layer.

A release liner web 16 is arranged on the adhesive skin contact layer 5 and over the release liner strips 15. The release liner web 16 is essentially co-extensive with the medical dressing web 100, as seen in the CD direction and is provided with a plurality of medical dressing specific markings 2.

The medical dressing specific markings 2 are arranged in a first and second row extending in a Cross Machine Direction CD, thereby defining a first and a second row 8,9 of medical dressing substrates 10, each of the first and a second row 8,9 of medical dressing substrates 10 being arranged between the first and the second longitudinal edge region 6a,7a of the medical dressing web 100 and in parallel as seen in the MD direction.

The medical dressing substrates 10 of the first and the second row 8,9 each has a first and a second longitudinal edge 11,12 extending in the MD direction and a first and second transverse edge 13,14 extending in the CD direction. The release liner strips 15 overlaps with the first longitudinal edges 11 of the medical dressing substrates 10. The edges, i.e. the contour of the medical dressing substrate may be indicated by a contour marking or may be predetermined and correlating to the position of the medical dressing specific markings.

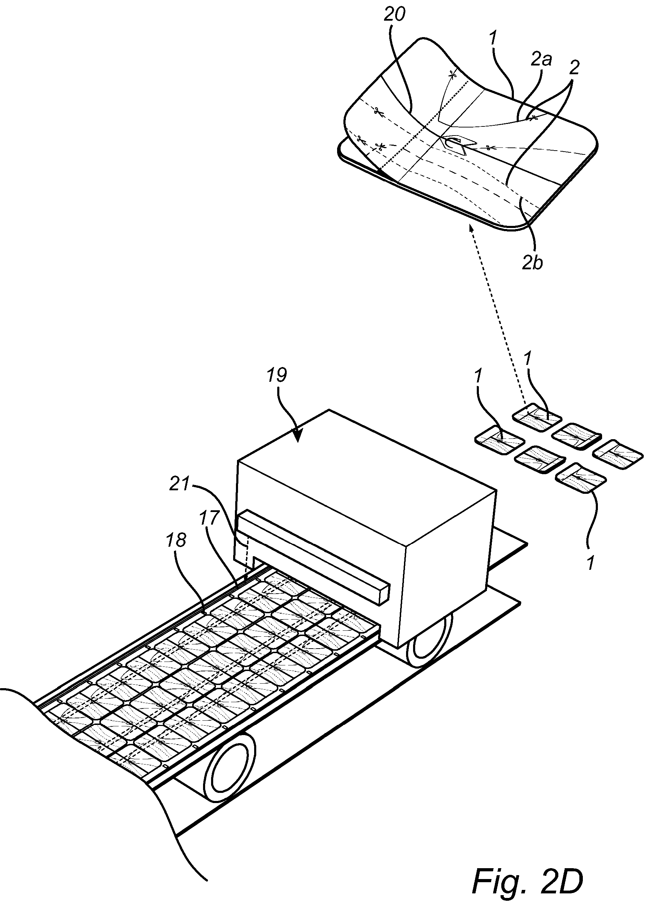

Since each one of the individual medical dressings comprises medical dressing specific markings 2, these needs to be identified prior to cutting out individual medical dressings 1 (shown in Fig. 2D). Furthermore, when applying the release liner web 16, the release liner web needs to be steered and positioned in relation to the medical dressing web 100 comprising the backing layer 2, the absorbent layer 4 and the adhesive skin contacting layer 5 and for positioning of the medical dressing specific markings 2.

The medical dressing specific markings 2 mean any type of markings preferably indicating a correct use or application of the dressing. For example, the medical dressing specific markings 2 may indicate how to cut, fold, use or apply the medical dressings. The markings are visually recognizable to the caregiver. The markings may be formed by a print pattern, a weld pattern, a compressed pattern, an embossed pattern, a material deposition pattern, or combinations thereof. Typically, the markings are printed or embossed. If the markings are embossed, the markings are also recognizable by touching. The markings in Fig. 1 are printed on the first release liner web. The markings may alternatively, or additionally, be embossed. The release liner web has a first surface facing the adhesive skin contact layer and a second opposing surface. The second surface faces the viewer. Typically, when the markings are formed by printing, the markings are printed onto the second surface of the release liner web 16.

The release liner web 16 comprises a CD synchronization mark 17 extending along the first and/or second longitudinal edge 6,7 of the continuous medical dressing web 100. As seen in the figure, the CD synchronization mark 18 is a continuous synchronization mark, extending continuously along the first longitudinal edge 6 of the medical dressing web 100 and in the first longitudinal edge region 6a, when the release liner 16 has been positioned on the medical dressing web 100 and forms part of the medical dressing web 100.

The release liner web 16 is furthermore provided with MD synchronization marks 18. The MD synchronization marks 18 is in these Figures aligned with the first transverse edges 13, here corresponding to the frontmost edges, of the medical dressing substrates 10 in the respective first and second row 8,9, as seen in the MD direction. However, the release liner web 16 may comprise a respective MD synchronization mark 18 aligned with the first and the second row 8,9 of the medical dressing substrates 10 such that the positions of the medical dressing specific markings 2 may be determined, as seen in the MD direction.

In figs. 1a and 1b the medical dressing substrates 10 of the first and the second row 8,9 each has a length l_{mds}, as measured in the MD direction and each of the medical dressing substrates 10 of the first and the second row 8,9 has the same length l_{mds}.

The respective MD synchronization mark 18 has a frontmost edge 18a, as seen in the MD direction, and a distance d₁₈ₐ between the frontmost edges 18a of the respective MD synchronization mark 18 of the first and second row 8,9, as measured in the MD direction, is the same as the length l_{mds} of the medical dressing substrates 10 of the first and the second row 8,9. This allows the distance between the frontmost edges 18a to be read and by predetermining the position of the respective MD synchronization mark 18 in relation to the medical dressing substrates 10 of the first and the second row 8,9 a position of the medical dressing specific markings 2 may be determined, as seen in the CD direction.

The release liner strips 15 are typically folded (see figure 1b). The folded portion forms a gripping tab that the applicator can grasp after the release liner web has been removed or displaced from its covering position.

The release liner web 16 may be configured to cover from 50 to 80 % e.g. from 60 to 70 % of the surface are of the adhesive skin contact layer 5. Accordingly, the area of the release liner web 16 overlapping the release liner strips 15 is from 20 to 50 %, e.g. from 30 to 40%. In Fig. 1, the release liner web 16 is co-extensive with the backing layer 3, however this is an optional feature.

The release liner web 16 and the release liner strips 15 may be formed from different materials.

Typically, the release liner web 16 comprises a first polymeric film, and wherein the release liner strips 15 comprise a second polymeric film, wherein the first polymeric film is different from the second polymeric film.

A polymeric film is beneficial since it allows for printing, welding, compressing and/or embossing the markings onto the release liner web 16.

The different materials; i.e. polymeric films of the release liner web 16 and the release liner strips 15 prevents undesired fusion or adherence between the two release liners in the area where the release liner web 16 overlaps with the release liner strips 15. Accordingly, removal of the release liner web 16 from the release liner strips (and from the dressing) is facilitated.

The first and/or the second polymeric film may be selected from a polyethylene film, a polypropylene film and a polyurethane film.

The release liner web 16 and/or the release liner strips 15 may consist of the polymeric film or may be a laminate comprising the polymeric film.

The thickness of the first and/or the second polymeric film is typically in the range of from 20 to 150 µm, e.g. from 40 to 120 µm, e.g. from 70 to 100 µm.

The release liner web 16 preferably comprises a polyethylene or a polypropylene film having a thickness in the range of from 40 to 140 µm, e.g. from 70 to 100 µm

Suitably, the first polymeric film is a polyethylene film and the second polymeric film is a polypropylene film.

The absorbent layer 4 preferably comprises a pressure-relieving material. If the dressing is to be used in conjunction with a medical device; i.e. for the purpose of alleviating medical device related pressure ulcers (MDRPU), an improved pressure-relieving effect may thus be obtained, and undesired scarfing caused by the medical device is avoided.

The absorbent layer 4 is not limited to a specific absorbent material, but preferably allows for the dressing to be cut through and yielding a pressure-relieving effect.

For example, the absorbent layer 4 may comprise a hydrophilic and/or absorbent foam. Preferably, the absorbent layer 4 comprises a polyurethane foam.

The absorbent layer 4 may comprise one or more sub-layers. If the absorbent layer 4 comprises a plurality of layers, the layers may be laminated or attached to each other to facilitate cutting through the dressing. Preferably, the absorbent layer 4 is void of superabsorbent particles or gel forming fibers, which may "leak" from the dressing during cutting.

The thickness of the absorbent layer 4 may be in the range of from 1 to 5 mm, e.g. from 2 to 3 mm. If the thickness of the absorbent layer 4 is too large, it may become difficult to fold and/or cut through the dressing.

The backing layer 3 may be adhesively attached to the absorbent layer 4. Alternatively, the backing layer 3 may be laminated to the absorbent layer 4. For example, heat lamination may be utilized to apply the backing layer 3 to the absorbent layer 4.

As used herein, the term "adhesive skin contact layer" means a layer configured to detachably adhere the dressing to a dermal surface. In other words, the adhesive skin contact layer is configured to contact the skin of a wearer.

Preferably, the adhesive skin contact layer 4 comprises a silicone-based adhesive. A silicone-based adhesive is gentle to the skin and may be removed from the skin without causing any trauma. For example, the adhesive skin contact layer 4 may comprise a silicone gel. The silicone gel may be provided as a coating on the absorbent layer.

The adhesive skin contact layer 4 may comprise one or more sub-layers. For example, the adhesive skin contact layer may comprise a polymeric film and an adhesive silicone gel layer, wherein the adhesive silicone gel layer is arranged to contact the skin.

The first transverse edges 13 of the medical dressing substrates 10 correspond in this figure to the frontmost edges of the medical dressing substrates 10, as seen in the MD direction, and the MD synchronization marks 17 are, as illustrated in Fig. 1, aligned with first transverse edges 13,14 of the medical dressing substrates 10 in the respective first and second row 8,9. Either the first transverse edges 13 may be marked on the release liner web, as illustrated in this figure, or they may be predetermined, but not marked out, in relation to the medical dressing specific marking 2.

The MD and CD synchronization marks are furthermore arranged in the first and/or second longitudinal edge region 6a,7a of the continuous medical dressing web 100. In Figures 1a and 1b, the MD and CD synchronization marks are both arranged in the first longitudinal edge region 6a,7a. The first and or the second edge regions 6a,7a may correspond to the outermost 25 mm of the web 100, as seen in the CD direction.

Each of the medical dressing substrates 10 has a transverse extension x, extending in the CD direction, and a longitudinal extension y, extending in the MD direction, wherein each of the medical dressing substrates 10 comprises a transverse center line Cₜ dividing the each of the medical dressing substrates 10 into a first portion 10a and a second portion 10b. The first 10a and the second 10b portion are symmetric about a transverse center line Cₜ of the respective medical dressing substrate 10, wherein the first portion 10a comprises a first set of medical dressing specific markings 2a, and wherein the second portion 10b comprises a second set of medical dressing specific markings 2b, and wherein the first set of markings 2a is different from the second set of markings 2b.

The first set of medical dressing specific markings 2a may be a first set of guiding lines indicating a correct use, application, cutting or folding of the medical dressing 1 dressing that is adapted for a first specific care situation. The second set of medical dressing specific markings 2b may be second set of guiding lines indicating a correct use, application, cutting or folding of the medical dressing that is adapted for a second, different care situation.

The individual medical dressings 1 (shown in Figure 2D) obtained after cutting the first and/or the second set of guiding lines may have a shape configured to mitigate pressure points from at least one medical device. Each of the cut-out medical dressing may be adapted to fit a particular facial area and to fit with a specific medical device.

The guiding lines of the first set of guiding lines may be adapted to yield dressing pieces adapted for a first facial area, and the second set of guiding lines may be adapted to yield dressing pieces adapted for a second facial area.

The term facial area is to be interpreted broadly. The "facial area" includes all parts of the patient's face, such as the cheeks, nose, nose ridge, forehead, the area between the mouth and the nose, and also includes the chin, neck and the area behind the ears. The transverse center line Cₜ may be indicated with at least one marking comprising a fold marking 20 indicating that the medical dressing should be folded prior to cutting.

The fold marking 20 may be formed by a print pattern, a weld pattern, a compressed pattern, an embossed pattern, a material deposition pattern, or combinations thereof.

Typically, the fold marking 20 is a printed marking.

The fold marking 20 is not limited to a specific shape, but any marking that guides the caregiver to fold the dressing may be utilized.

In figures 1a and 1b, the fold marking 20 comprises a curved arrow, which serves to emphasize that the medical dressing should be folded. Accordingly, the staff or caregivers are directed to folding the dressing prior to cutting the dressing into pieces.

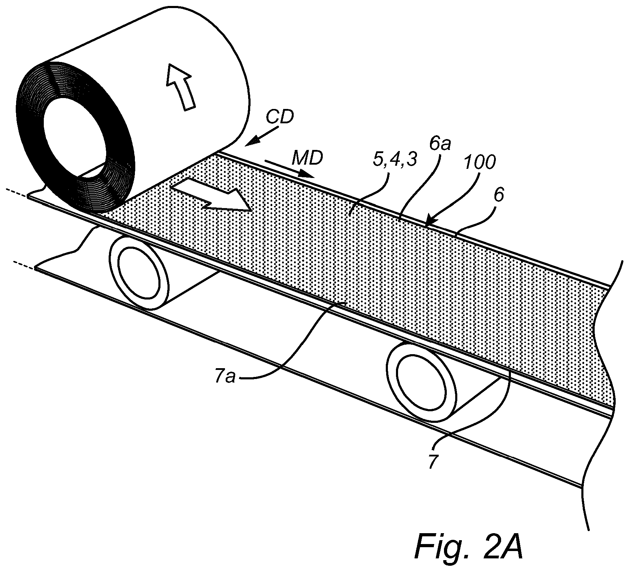

A method for manufacturing medical dressings 1 comprising medical dressing specific markings 2 according to the present disclosure is shown in Figures 2A to 2D.

In a first step, illustrated in figure 2A, a continuous medical dressing web 100 is fed in a Machine Direction MD. The medical dressing web 100 comprises a backing layer 3, an absorbent layer 4 and an adhesive skin contact layer 5, the absorbent layer 4 being arranged between the backing layer 3 and the adhesive skin contact layer 5. The continuous medical dressing web 100 has a first and a second longitudinal edge 6,7 extending in the Machine Direction MD and a first and a second longitudinal edge region 6a,7a extending along the first and the second longitudinal edge 6,7 of the continuous medical dressing web 100.

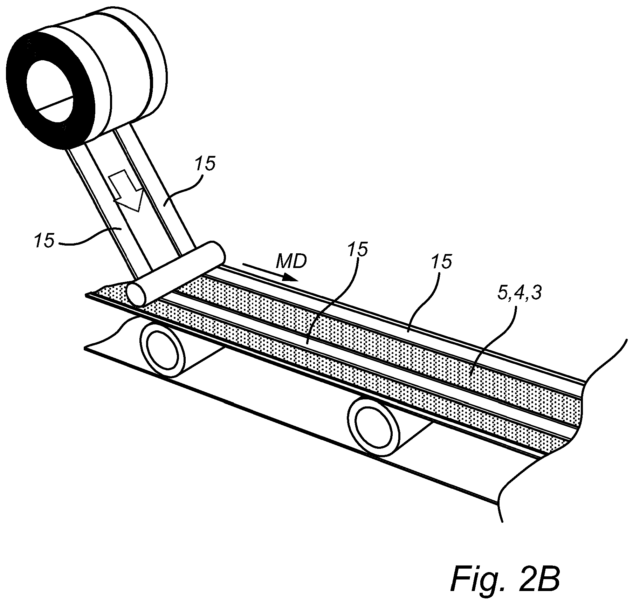

In figure 2B, an optional second step of arranging release liner strips 15 onto the adhesive skin contact layer 5 is illustrated.

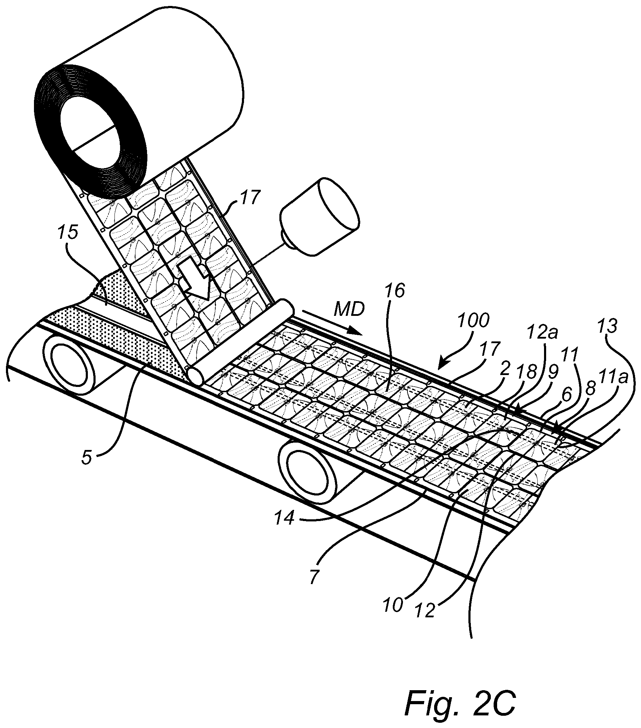

Figure 2C illustrates application of a release liner web 16 onto the adhesive skin contact layer 5. The release liner web 16 is essentially co-extensive with the medical dressing web 100 and is provided with a plurality of medical dressing specific markings 2. The plurality of medical dressing specific markings 2 are arranged in a first and second row extending in a Cross Machine Direction CD, thereby defining a first and a second row 8,9 of medical dressing substrates 10, each of the first and a second row 8,9 of medical dressing substrates 10 being arranged between the first and the second longitudinal edge region 6a,7a of the medical dressing web 100 and in parallel as seen in the MD direction.

The medical dressing substrates 10 of the first and the second row 8,9 each has a first and a second longitudinal edge 11,12 extending in the MD direction and a first and second transverse edge 13,14 extending in the CD direction.

The release liner strips 15 overlap with the first longitudinal edges 11 of the medical dressing substrates 10. The edges, i.e. the contour of the medical dressing substrate may be indicated by a contour marking or may be predetermined and correlating to the position of the medical dressing specific markings.

The release liner web 16 furthermore comprises CD synchronization marks 17 and MD synchronization marks 18.

The continuous CD synchronization mark 17 extends along the first longitudinal edge 6 of the continuous medical dressing web 100 and is arranged in the first longitudinal edge portion 6a of the web 100.

The MD synchronization marks 18 are aligned with the first transverse edges 13, here the frontmost edges, of the medical dressing substrates 10 in the respective first and second row 8,9, as seen in the MD direction.

Prior to applying the release liner web 16, the CD synchronization mark 17 on the release liner web 16 is detected to determine the position of the release liner web 16 and if an adjustment of the release liner web 16 and/or the medical dressing web 100 is needed to align the release liner web 16 and the medical dressing web 100 such that they are aligned as seen in the CD direction.

Figure 2D illustrates the steps of feeding the continuous medical dressing web 100 towards a contour cutting tool 19 and detecting the respective MD synchronization marks 18 to align the cutting tool 19 with the first and the second rows 8,9 of medical dressing substrates 10 as defined by the medical dressing specific markings 2 consequently and cutting out individual medical dressings 1 from the continuous medical dressing web 100 row-by-row.

To detect the MD synchronization marks 18, the continuous medical dressing web 100 passes a detecting sensor 21.

The detecting sensor 21 detects the position of each of the MD synchronization marks 18. The position of the MD synchronization marks 18 triggers a positioning of the contour cutting tool 19 to align the contour cutting tool 19 such that the cutting fit with the layout of the medical dressing specific markings 2. Individual medical dressings 1 are cut out, one row at a time, from the continuous medical dressing web 100 with the contour cutting tool 19.

The contour cutting tool 19 may comprise a rotary cutting tool and a cutting counter roller, such as vertical Rotary Die Cutter (RDC), a laser cutting tool or a flat bed punching tool.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for manufacturing medical dressings (1) comprising medical dressing specific markings (2), the method comprising the following steps;
- feeding a continuous medical dressing web (100) in a Machine Direction (MD), the medical dressing web (100) comprising a backing layer (3), an absorbent layer (4) and an adhesive skin contact layer (5), the absorbent layer (4) being arranged between the backing layer (3) and the adhesive skin contact layer (5) the continuous medical dressing web (100) having a first and a second longitudinal edge (6,7) extending in the Machine Direction (MD) and a first and a second longitudinal edge region (6a,7a) extending along the first and the second longitudinal edge (6,7) of the continuous medical dressing web (100);
- providing a release liner web (16), the release liner web (16) being provided with a plurality of medical dressing specific markings (2) arranged in a first and second row extending in the Cross machine Direction (CD), thereby defining a first and a second row (8,9) of medical dressing substrates (10) comprising a respective medical dressing specific marking (2), each of the first and the second row (8,9) of medical dressing substrates (10) being arranged between the first and the second longitudinal edge region (6a,7a) of the medical dressing web (100), the release liner web (16) furthermore being provided with a continuous or discontinuous CD synchronization mark (17) extending along the first and/or second longitudinal edge (6,7) of the continuous medical dressing web (1), the release liner web (16) furthermore being provided with a respective MD synchronization mark (18) for the first and the second row of medical dressing substrates (8,9);
- detecting the CD synchronization mark(s) (17) on the release line web (16) to align the release line web (16) with the medical dressing web (100), as seen in the CD direction;
- arranging the release liner web (16) onto the adhesive skin contact layer (5);
- detecting the respective MD synchronization mark (18) and positioning a contour cutting tool (19) in a predetermined position with respect to the first and the second rows (8,9) of medical dressing substrates (10), defined by the first and second row of medical dressing specific markings (2), based on the position of the detected respective MD synchronization mark (18); and
- cutting out individual medical dressings (1) from the continuous medical dressing web (100) with the contour cutting tool (19).

2. The method for manufacturing medical dressings (1) according to claim 1, wherein the medical dressing substrates (10) of the first and the second row (8,9) each has a length (l_{mds}), as measured in the MD direction and wherein the respective MD synchronization mark (18) has a frontmost edge (18a), as seen in the MD direction, and wherein a distance (d₁₈ₐ) between the frontmost edge (18a) of the respective MD synchronization mark (18) of the first row and the frontmost edge (18a) of the MD synchronization mark (18) of the second row, as measured in the MD direction, is the same as the length (l_{mds}) of the medical dressing substrates (10) of the first and the second row (8,9).

3. The method for manufacturing medical dressings according to claim 2, wherein each of the medical dressing substrates (10) of the first and the second row (8,9) has the same length (l_{mds}).

4. The method for manufacturing medical dressings according to any one of the preceding claims, wherein the method comprises a step of;
- arranging one or more release liner strips (15) onto the adhesive skin contact layer (5), and in a subsequent step;
- arranging the release liner web (16) on top the one or more release liner strips (15) and on the adhesive skin contact layer (5).

5. The method for manufacturing medical dressing according to claim 4, wherein the release liner web (16) and the release liner strips (15) are formed from different materials.

6. The method for manufacturing medical dressing according to claim 4 or 5, wherein the step of arranging one or more release liner strips (15) includes arranging one or more continuous release liner strip webs onto the adhesive skin contact layer (5).

7. The method for manufacturing medical dressing according to any one of claims 2 to 4, wherein the release liner web (16) comprises a first polymeric film, and wherein the release liner strips (15) comprises a second polymeric film, wherein said first polymeric film is different from said second polymeric film.

8. The method for manufacturing medical dressing according to claim 7, wherein said first and/or said second polymeric film is selected from a polyethylene film, a polypropylene film and a polyurethane film.

9. The method for manufacturing medical dressing according to any one of claims 4 to 8, wherein the one or more release liner strips (15) is/are folded release liner strip(s) comprising a respective folding line extending in the MD direction and overlapping with each of the medical dressing substrates (10) in the respective first and second row (8,9).

10. The method for manufacturing medical dressings (1) according to any one of the preceding claims, wherein the MD synchronization marks (17) are aligned with first transverse edges (13) of the medical dressing substrates (10) in the respective first and second row (8,9), optionally, the first transverse edges of the medical dressing substrates (10) in the respective first and second row (8,9) correspond to the frontmost edges, as seen in the MD direction.

11. The method for manufacturing medical dressings (1) according to any one of the preceding claims, wherein the MD and CD synchronization marks (17,18) are arranged in the first and/or second longitudinal edge region (6a,7a) of the continuous medical dressing web (100), preferably wherein the MC and CD synchronization marks are arranged in the same first and/or second longitudinal edge region (6a,7a).

12. The method for manufacturing medical dressings (1) according to any one of the preceding claims, wherein the medical dressing specific markings (2) are individual instructions indicating how to cut, fold, use or apply said dressings (1).

13. The method for manufacturing medical dressings (1) according to any one of the preceding claims, wherein the medical dressing specific markings (2) are formed by a print pattern, a weld pattern, a compressed pattern, an embossed pattern, a material deposition pattern, or combinations thereof.

## Patentansprüche

1. Verfahren zur Herstellung von medizinischen Verbänden (1), die spezifische Markierungen (2) für medizinische Verbände umfassen, wobei das Verfahren die folgenden Schritte umfasst:
- Zuführen einer durchgehenden Bahn (100) für medizinische Verbände in einer Maschinenrichtung (MD), wobei die Bahn (100) für medizinische Verbände eine Trägerschicht (3), eine absorbierende Schicht (4) und eine haftende Hautkontaktschicht (5) umfasst, wobei die absorbierende Schicht (4) zwischen der Trägerschicht (3) und der haftenden Hautkontaktschicht (5) angeordnet ist, wobei die durchgehende Bahn (100) für medizinische Verbände einen ersten und einen zweiten längs verlaufenden Rand (6, 7), der sich in der Maschinenrichtung (MD) erstreckt, und einen ersten und einen zweiten längs verlaufenden Randbereich (6a, 7a) aufweist, der sich entlang des ersten und des zweiten längs verlaufenden Rands (6, 7) der durchgehenden Bahn (100) für medizinische Verbände erstreckt;
- Bereitstellen einer Trennschichtbahn (16), wobei die Trennschichtbahn (16) mit einer Vielzahl von spezifischen Markierungen (2) für medizinische Verbände versehen ist, die in einer ersten und zweiten Reihe angeordnet sind, die sich in der Querrichtung (CD) erstrecken, wodurch eine erste und eine zweite Reihe (8, 9) von Substraten (10) für medizinische Verbände festgelegt werden, die eine jeweilige spezifische Markierung (2) für medizinische Verbände umfassen, wobei sowohl die erste als auch die zweite Reihe (8, 9) von Substraten (10) für medizinische Verbände zwischen dem ersten und dem zweiten längs verlaufenden Randbereich (6a, 7a) der Bahn (100) für medizinische Verbände angeordnet ist, wobei die Trennschichtbahn (16) ferner mit einer kontinuierlichen oder unterbrochenen CD-Abgleichmarke (17) versehen ist, die sich entlang des ersten und/oder zweiten längs verlaufenden Rands (6, 7) der durchgehenden Bahn (1) für medizinische Verbände erstreckt, wobei die Trennschichtbahn (16) ferner mit einer jeweiligen MD-Abgleichmarke (18) für die erste und die zweite Reihe von Substraten für medizinische Verbände (8, 9) versehen ist;
- Erkennen der CD-Abgleichmarke(n) (17) auf der Trennschichtbahn (16) zum Ausrichten der Trennschichtbahn (16) nach der Bahn (100) für medizinische Verbände bei Betrachtung in der Richtung CD;
- Anordnen der Trennschichtbahn (16) auf der haftenden Hautkontaktschicht (5);
- Erkennen der jeweiligen MD-Abgleichmarke (18) und Platzieren eines Umrissschneidwerkzeugs (19) in einer vorher festgelegten Position bezogen auf die erste und die zweite Reihe (8, 9) von Substraten (10) für medizinische Verbände, die durch die erste und zweite Reihe von spezifischen Markierungen (2) für medizinische Verbände festgelegt sind, auf Grundlage der Position der erkannten jeweiligen MD-Abgleichmarke (18); und
- Ausschneiden von einzelnen medizinischen Verbänden (1) aus der durchgehenden Bahn (100) für medizinische Verbände mit dem Umrissschneidwerkzeug (19).

2. Verfahren zur Herstellung von medizinischen Verbänden (1) nach Anspruch 1, wobei die Substrate (10) für medizinische Verbände der ersten und der zweiten Reihe (8, 9) jeweils eine in der Richtung MD gemessene Länge (l_{mds}) aufweisen und wobei die jeweilige MD-Abgleichmarke (18) in der Richtung MD betrachtet eine vorderste Kante (18a) aufweist und wobei ein in der Richtung MD gemessener Abstand (d₁₈ₐ) zwischen der vordersten Kante (18a) der jeweiligen MD-Abgleichmarke (18) der ersten Reihe und der vordersten Kante (18a) der MD-Abgleichmarke (18) der zweiten Reihe identisch ist mit der Länge (I_{mds}) der Substrate (10) für medizinische Verbände der ersten und der zweiten Reihe (8, 9).

3. Verfahren zur Herstellung von medizinischen Verbänden nach Anspruch 2, wobei jedes der Substrate (10) für medizinische Verbände der ersten und der zweiten Reihe (8, 9) die gleiche Länge (I_{mds}) aufweist.

4. Verfahren zur Herstellung von medizinischen Verbänden nach einem der vorhergehenden Ansprüche, wobei das Verfahren folgenden Schritt umfasst:
- Anordnen von einem oder mehreren Trennschichtstreifen (15) auf der haftenden Hautkontaktschicht (5), und in einem nachfolgenden Schritt:
- Anordnen der Trennschichtbahn (16) oben auf dem einen oder den mehreren Trennschichtstreifen (15) und auf der haftenden Hautkontaktschicht (5).

5. Verfahren zur Herstellung eines medizinischen Verbands nach Anspruch 4, wobei die Trennschichtbahn (16) und die Trennschichtstreifen (15) aus verschiedenen Materialien gebildet sind.

6. Verfahren zur Herstellung eines medizinischen Verbands nach Anspruch 4 oder 5, wobei der Schritt des Anordnens von einem oder mehreren Trennschichtstreifen (15) Anordnen von einer oder mehreren durchgehenden Bahnen für Trennschichtstreifen auf der haftenden Hautkontaktschicht (5) umfasst.

7. Verfahren zur Herstellung eines medizinischen Verbands nach einem der Ansprüche 2 bis 4, wobei die Trennschichtbahn (16) eine erste Polymerfolie umfasst und wobei der Trennschichtstreifen (15) eine zweite Polymerfolie umfasst, wobei sich die erste Polymerfolie von der zweiten Polymerfolie unterscheidet.

8. Verfahren zur Herstellung eines medizinischen Verbands nach Anspruch 7, wobei die erste und/oder die zweite Polymerfolie aus einer Polyethylenfolie, einer Polypropylenfolie und einer Polyurethanfolie ausgewählt ist.

9. Verfahren zur Herstellung eines medizinischen Verbands nach einem der Ansprüche 4 bis 8, wobei der eine oder die mehreren Trennschichtstreifen (15) ein gefalteter/gefaltete Trennschichtstreifen ist bzw. sind, die eine jeweilige Falzlinie umfassen, die sich in der Richtung MD erstreckt und sich mit jedem der Substrate (10) für medizinische Verbände in der jeweiligen erste und zweiten Reihe (8, 9) überlappt.

10. Verfahren zur Herstellung von medizinischen Verbänden (1) nach einem der vorhergehenden Ansprüche, wobei die MD-Abgleichmarken (17) nach ersten quer verlaufenden Rändern (13) der Substrate (10) für medizinische Verbände in der jeweiligen ersten und zweiten Reihe (8, 9) ausgerichtet werden, wobei die ersten quer verlaufenden Ränder der Substrate (10) für medizinische Verbände in der jeweiligen ersten und zweiten Reihe (8, 9) optional den in der Richtung MD betrachtet vordersten Kanten entsprechen.

11. Verfahren zur Herstellung von medizinischen Verbänden (1) nach einem der vorhergehenden Ansprüche, wobei die MD- und CD-Abgleichmarken (17, 18) im ersten und/oder zweiten längs verlaufenden Randbereich (6a, 7a) der durchgehenden Bahn (100) für medizinische Verbände angeordnet sind, wobei vorzugsweise die MD- und CD-Abgleichmarken im selben ersten und/oder zweiten längs verlaufenden Randbereich (6a, 7a) angeordnet sind.

12. Verfahren zur Herstellung von medizinischen Verbänden (1) nach einem der vorhergehenden Ansprüche, wobei die spezifischen Markierungen (2) für medizinische Verbände individuelle Anweisungen dafür sind, wie die Verbände (1) zugeschnitten, gefaltet oder angewendet werden sollen.

13. Verfahren zur Herstellung von medizinischen Verbänden (1) nach einem der vorhergehenden Ansprüche, wobei die spezifischen Markierungen (2) für medizinische Verbände von einem Druckmuster, einem Schweißmuster, einem eingedrückten Muster, einem Prägemuster, einem Materialauftragsmuster oder Kombinationen daraus gebildet werden.

## Revendications

1. Procédé de fabrication de pansements médicaux (1) comprenant des marquages spécifiques de pansements médicaux (2), le procédé comprenant les étapes suivantes :
- apport d'une bande continue de pansement médical (100) dans le sens machine (MD), la bande continue de pansement médical (100) comprenant une couche support (3), une couche absorbante (4) et une couche adhésive de contact avec la peau (5), la couche absorbante (4) étant disposée entre la couche support (3) et la couche adhésive de contact avec la peau (5), la bande continue de pansement médical (100) ayant un premier et un second bord longitudinal (6,7) s'étendant dans le sens machine (MD) et une première et une seconde zone de bord longitudinal (6a, 7a) s'étendant le long du premier et du second bord longitudinal (6,7) de la bande continue de pansement médical (100) ;
- prévision d'une bande de revêtement antiadhésif (16), la bande de revêtement antiadhésif (16) étant pourvue d'une pluralité de marquages spécifiques de pansement médical (2) disposés en une première et une seconde rangée s'étendant dans le sens travers de la machine (CD), en définissant ainsi une première et une seconde rangée (8,9) de substrats de pansement médical (10) comprenant un marquage spécifique de pansement médical respectif (2), chacune des première et seconde rangées (8,9) de substrats de pansement médical (10) étant disposée entre la première et la seconde zone de bord longitudinal (6a, 7a) de la bande de pansement médical (100), la bande de revêtement antiadhésif (16) étant en outre pourvue d'une marque de synchronisation CD continue ou discontinue (17) s'étendant le long du premier et/ou du second bord longitudinal (6,7) de la bande de pansement médical continue (1), la bande de revêtement antiadhésif (16) étant en outre pourvue d'une marque de synchronisation MD respective (18) pour la première et la seconde rangée de substrats de pansement médical (8,9) ;
- détection de la ou des marques de synchronisation CD (17) sur la bande de six (16) pour aligner la bande de revêtement antiadhésif (16) avec la bande de pansement médical (100), vu dans le sens CD ;
- disposition de la bande de revêtement antiadhésif (16) sur la couche adhésive de contact avec la peau (5) ;
- détection de la marque de synchronisation MD respective (18) et positionnement d'un outil de coupe de contour (19) dans une position prédéterminée par rapport aux première et seconde rangées (8, 9) de substrats de pansements médicaux (10), définie par des première et seconde rangées de marques spécifiques de pansements médicaux (2), sur la base de la position de la marque de synchronisation MD respective détectée (18) ; et
- découpage de pansements médicaux individuels (1) dans la bande continue de pansement médical (100) à l'aide de l'outil de coupe de contour (19).

2. Procédé de fabrication de pansements médicaux (1) selon la revendication 1, dans lequel les substrats de pansement médical (10) de la première et de la deuxième rangée (8, 9) ont chacun une longueur (l_{mds}), mesurée dans la direction MD, et la marque de synchronisation MD respective (18) a un bord le plus en avant (18a), vu dans la direction MD, et une distance (d₁₈ₐ) entre le bord le plus en avant (18a) de la marque de synchronisation MD respective (18) de la première rangée et le bord le plus en avant (18a) de la marque de synchronisation MD (18) de la seconde rangée, mesurée dans la direction MD, est la même que la longueur (l_{mds}) des substrats de pansement médical (10) de la première et de la seconde rangée (8, 9).

3. Procédé de fabrication de pansements médicaux selon la revendication 2, dans lequel chacun des substrats de pansement médical (10) de la première et de la seconde rangée (8, 9) a la même longueur (l_{mds}).

4. Procédé de fabrication de pansements médicaux selon l'une quelconque des revendications précédentes, le procédé comprenant une étape consistant à :
- disposer une ou plusieurs languettes de revêtement antiadhésif (15) sur la couche adhésive de contact avec la peau (5) et, dans une étape ultérieure ;
- disposer la bande de revêtement antiadhésif (16) sur la ou les languettes de revêtement antiadhésif (15) et sur la couche adhésive de contact avec la peau (5).

5. Procédé de fabrication d'un pansement médical selon la revendication 4, dans lequel la bande de revêtement antiadhésif (16) et les languettes de revêtement antiadhésif (15) sont formées de matériaux différents.

6. Procédé de fabrication d'un pansement médical selon la revendication 4 ou 5, dans lequel l'étape de disposition d'une ou plusieurs languettes de revêtement antiadhésif (15) comprend la disposition d'une ou plusieurs bandes continues de bandes de revêtement antiadhésif sur la couche adhésive de contact avec la peau (5).

7. Procédé de fabrication d'un pansement médical selon l'une quelconque des revendications 2 à 4, dans lequel la bande de revêtement antiadhésif (16) comprend un premier film polymère, et les languettes de revêtement antiadhésif (15) comprennent un second film polymère, ledit premier film polymère étant différent dudit second film polymère.

8. Procédé de fabrication d'un pansement médical selon la revendication 7, dans lequel ledit premier et/ou ledit second film polymère est choisi parmi un film de polyéthylène, un film de polypropylène et un film de polyuréthane.

9. Procédé de fabrication d'un pansement médical selon l'une quelconque des revendications 4 à 8, dans lequel la ou les languettes de revêtement antiadhésif (15) est/sont une ou des bandes de revêtement antiadhésif pliées comprenant une ligne de pliage respective s'étendant dans la direction MD et chevauchant chacun des substrats de pansement médical (10) dans les première et seconde rangées respectives (8, 9).

10. Procédé de fabrication de pansements médicaux (1) selon l'une quelconque des revendications précédentes, dans lequel les marques de synchronisation MD (17) sont alignées avec des premiers bords transversaux (13) des substrats de pansement médical (10) dans les première et seconde rangées respectives (8, 9), facultativement, les premiers bords transversaux des substrats de pansement médical (10) dans les première et seconde rangées respectives (8, 9) correspondant aux bords les plus avant, comme vu dans la direction MD.

11. Procédé de fabrication de pansements médicaux (1) selon l'une quelconque des revendications précédentes, dans lequel les marques de synchronisation MD et CD (17, 18) sont disposées dans la première et/ou la seconde zone de bord longitudinal (6a, 7a) de la bande continue de pansement médical (100), les marques de synchronisation MC et CD étant de préférence disposées dans la même première et/ou seconde zone de bord longitudinal (6a, 7a).

12. Procédé de fabrication de pansements médicaux (1) selon l'une quelconque des revendications précédentes, dans lequel les marquages spécifiques de pansements médicaux (2) sont des instructions individuelles indiquant comment couper, plier, utiliser ou appliquer lesdits pansements (1).

13. Procédé de fabrication de pansements médicaux (1) selon l'une quelconque des revendications précédentes, dans lequel les marquages spécifiques de pansements médicaux (2) sont formés par un motif imprimé, un motif de soudure, un motif comprimé, un motif gaufré, un motif à dépôt de matériau, ou des combinaisons de ceux-ci.
